# EUROPEAN PATENT APPLICATION

(11) **EP 4 631 519 A1**
(43) Date of publication of application: **15.10.2025**
(21) Application number: 25170219.7
(22) Date of filing: 11.04.2025
(51) Int. Cl.: A61K 38/16, A61K 36/42, A61P 3/08, A61P 3/10

(54) **COMPOSITION FOR USE IN LOWERING BLOOD GLUCOSE LEVEL**

(30) Priority: 12.04.2024 TW 113113817
(71) Applicant: Baosheng Biomedical Research Co., Ltd., Taichung City 404 (TW); Biotrue Co., Ltd., Taichung City 411 (TW); Wu, Wen-Liang, Taichung City 404 (TW)
(72) Inventor: HUANG, Guan-Jhong, 404 TAICHUNG CITY (TW)
(74) Representative: Regimbeau

(57) **Abstract**

A composition containing a *Momordica* charantia-derived exosome and a *Momordica charantia* insulin receptor-binding protein for use in lowering blood glucose level in a subject in need thereof. A weight ratio of the *Momordica* charantia-derived exosome to the *Momordica charantia* insulin receptor-binding protein ranges from 2:1 to 5:1. The composition is a food product or a pharmaceutical composition.

## Description

The Sequence Listing submitted concurrently herewith with a file name of "PE-71833-EU-SEQUENCE LISTING.xml," a creation date of March 27, 2025, and a size of 2.05 kilobytes, is part of the specification and is incorporated by reference in its entirety.

The disclosure relates to a method for lowering blood glucose level using a composition containing a *Momordica* charantia-derived exosome and a *Momordica charantia* insulin receptor-binding protein.

Regulation of blood glucose level (i.e., a glucose concentration in a blood) is a crucial part for maintaining metabolic homeostasis. A prolonged state of excessively high glucose level may lead to the onset of glucose toxicity, resulting in dysregulation of insulin-producing pancreatic β cells, and even causing diabetes mellitus (DM) and complications thereof, which includes cardiovascular disease, chronic kidney disease, foot ulcers, and diabetic ketoacidosis.

At present, drugs used clinically to lower blood glucose level may include biguanides (such as metformin), sulfonylureas, and meglitinides. However, the effects of the aforesaid drugs are not satisfactory, and use thereof may cause severe side effects and adverse effects to patients. Therefore, researchers in relevant fields strive to find a natural active component from a plant that can be used to lower blood glucose level.

In *"*Research and Development of Insulin Receptor Targeting Antidiabetic Drug Based on Experiences of Traditional Chinese Medicine" (PhD thesis by Hsin-Yi Lo (2014) (hereinafter "Lo"); Graduate Institute of Chinese Medicine, China Medical University, Taiwan), Lo subjected *Momordica charantia* seeds to an extraction treatment, and found that an extract of the *Momordica charantia* seeds thus obtained contained a protein having hypoglycemic activity, that is, *Momordica charantia* insulin receptor-binding protein (mcIRBP). Lo had proved through *in vivo* experiments that the mcIRBP could effectively lower blood glucose level in normal mice and diabetic mice.

In spite of the aforesaid, there is still a need for those skilled in the art to develop a composition which contains at least the mcIRBP, so as to further exert the hypoglycemic effect of the mcIRBP.

Accordingly, an object of the present disclosure is to provide a composition for use in lowering blood glucose level, which can alleviate at least one of the drawbacks of the prior art.

According to an aspect of the disclosure, there is provided the composition according to claim 1.

Other features and advantages of the disclosure will become apparent in the following detailed description of the embodiment(s) with reference to the accompanying drawings. It is noted that various features may not be drawn to scale.

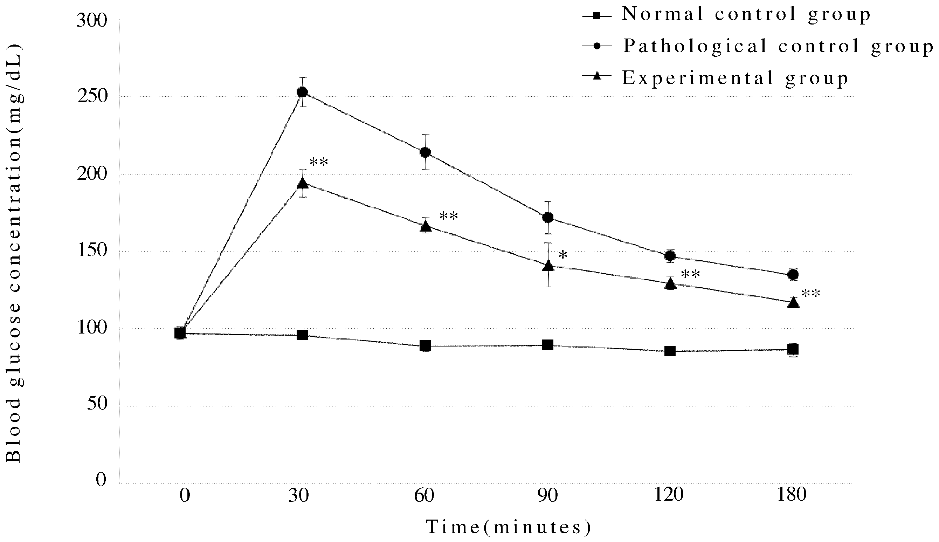

The sole figure shows a blood glucose concentration versus time curve of each group of Example 1, *infra,* in which the symbols "*" and "**" represent p<0.05 and p<0.01, respectively (compared with the pathological control group).

It is to be understood that, if any prior art publication is referred to herein, such reference does not constitute an admission that the publication forms a part of the common general knowledge in the art, in Taiwan or any other country.

For the purpose of this specification, it will be clearly understood that the word "comprising" means "including but not limited to", and that the word "comprises" has a corresponding meaning.

Unless otherwise defined, all technical and scientific terms used herein have the meaning commonly understood by a person skilled in the art to which the present disclosure belongs. One skilled in the art will recognize many methods and materials similar or equivalent to those described herein, which could be used in the practice of the present disclosure. Indeed, the present disclosure is in no way limited to the methods and materials described.

By conducting research, the applicants surprisingly found that a composition containing *Momordica charantia* exosomes and *Momordica charantia* insulin receptor-binding proteins (mcIRBP) showed excellent effects in reducing blood glucose level.

Accordingly, the present disclosure provides a composition containing a *Momordica charantia*-derived exosome and a *Momordica charantia* insulin receptor-binding protein for use in lowering blood glucose level in a subject in need thereof.

The present disclosure also provides a method for lowering blood glucose level, which includes administering to a subject in need thereof a composition containing a *Momordica* charantia-derived exosome and a *Momordica charantia* insulin receptor-binding protein (mcIRBP). In certain embodiments, the subject may have type 1 diabetes mellitus (T1DM) or type 2 diabetes mellitus (T2DM).

As used herein, the terms "administering" and "administration" can be interchangeably used, and refer to introducing, providing or delivering a predetermined active ingredient to a subject by any suitable routes to perform its intended function.

As used herein, the term "subject" refers to any animal of interest, such as humans, monkeys, cows, sheep, horses, pigs, goats, dogs, cats, mice, and rats. In certain embodiments, the subject is a human.

According to the present disclosure, the *Momordica charantia-*derived exosome may be obtained from *Momordica charantia* using techniques well known to those skilled in the art. In this regard, those skilled in the art may refer to journal articles, e.g., Wang F. et al. (2023), Molecules, doi: 10.3390/molecules28176182.

According to the present disclosure, the *Momordica charantia-*derived exosome may be obtained by an extraction treatment selected from the group consisting of centrifugation (e.g., ultracentrifugation), filtration (e.g., ultrafiltration), dialysis, chromatography, and combinations thereof. In an exemplary embodiment, the extraction treatment is a combination of the centrifugation and the filtration.

According to the present disclosure, the *Momordica charantia* insulin receptor-binding protein may be a commercially available product, or may be prepared using a conventionally used chemical synthesis method or genetic engineering techniques well known to those skilled in the art. In an exemplary embodiment, preparation of the *Momordica charantia* insulin receptor-binding protein (NCBI Reference Sequence: XP_022137472.1) (SEQ ID NO: 1) is entrusted to Tri-I Biotech, Inc.

According to the present disclosure, the *Momordica charantia* insulin receptor-binding protein may be obtained from *Momordica charantia* using techniques well known to those skilled in the art. In this regard, those skilled in the art may refer to the literature, e.g., the PhD thesis by Lo (2014), *supra.*

In certain embodiments, a weight ratio of the *Momordica charantia-*derived exosome to the *Momordica charantia* insulin receptor-binding protein may range from 2:1 to 5:1. In an exemplary embodiment, the weight ratio of the *Momordica* charantia-derived exosome to the *Momordica charantia* insulin receptor-binding protein is 2:1.

According to the present disclosure, the *Momordica charantia-*derived exosome and the *Momordica charantia* insulin receptor-binding protein may be co-administered to a subject. That is to say, the *Momordica charantia-*derived exosome and the *Momordica charantia* insulin receptor-binding protein may be either administered together in a single dosage form or administered simultaneously in separate oral dosage forms. Alternatively, the *Momordica* charantia-derived exosome and the *Momordica charantia* insulin receptor-binding protein may be administered alternately or sequentially in separate dosage forms at a predetermined time interval. The length of the predetermined time interval can be adjusted as required, so as to allow pharmacological effects of the *Momordica* charantia-derived exosome and the *Momordica charantia* insulin receptor-binding protein to be exerted simultaneously or exerted not at the same time. In an exemplary embodiment, the *Momordica* charantia-derived exosome and the *Momordica charantia* insulin receptor-binding protein are administered simultaneously.

According to the present disclosure, the composition may be formulated as a food product using a standard technique well known to one of ordinary skill in the art. For example, the composition may be directly added to an edible material or may be used to prepare an intermediate composition (e.g., a premix) suitable to be subsequently added to the edible material.

As used herein, the term "food product" refers to any article or substance that can be ingested by a subject into the body thereof. Examples of the food product may include, but are not limited to, milk powders, fermented milk, yogurt, butter, beverages (e.g., tea, coffee, etc.), functional beverages, a flour product, baked foods, confectionery, candies, fermented foods, animal feeds, health foods, infant foods, and dietary supplements.

According to the present disclosure, the composition may be prepared in the form of a pharmaceutical composition. The pharmaceutical composition may be formulated into a dosage form suitable for parenteral administration, oral administration, or topical administration using technology well known to those skilled in the art.

According to the present disclosure, the pharmaceutical composition may further include a pharmaceutically acceptable carrier widely employed in the art of drug-manufacturing. For instance, the pharmaceutically acceptable carrier may include one or more of the following agents: solvents, buffers, emulsifiers, suspending agents, decomposers, disintegrating agents, dispersing agents, binding agents, excipients, stabilizing agents, chelating agents, diluents, gelling agents, preservatives, wetting agents, lubricants, absorption delaying agents, liposomes, and the like. The choice and amount of the aforesaid agents are within the expertise and routine skills of those skilled in the art.

For parenteral administration, the pharmaceutical composition according to the present disclosure may be formulated into an injection, e.g., a sterile aqueous solution or a dispersion.

The pharmaceutical composition according to the present disclosure may be administered via one of the following parenteral routes: intraperitoneal injection, intrapleural injection, intramuscular injection, intravenous injection, intraarterial injection, intraarticular injection, intrasynovial injection, intrathecal injection, intracranial injection, intraepidermal injection, subcutaneous injection, intradermal injection, intralesional injection, and sublingual administration.

According to the present disclosure, the dosage form suitable for oral administration includes, but is not limited to, sterile powders, tablets, troches, lozenges, pellets, capsules, dispersible powders or granules, solutions, suspensions, emulsions, syrup, elixir, slurry, and the like.

According to the present disclosure, the pharmaceutical composition may be formulated into an external preparation suitable for topical application to the skin using technology well known to those skilled in the art. The external preparation includes, but is not limited to, emulsions, gels, ointments, creams, patches, liniments, powder, aerosols, sprays, lotions, serums, pastes, foams, drops, suspensions, salves, and bandages.

According to the present disclosure, the dose and frequency of administration of the composition may vary depending on the following factors: the severity of the illness or disorder to be treated, routes of administration, and age, physical condition and response of the subject to be treated. In general, the composition may be administered in a single dose or in several doses. In certain embodiments, the composition may be administered one time or two times a day.

The disclosure will be further described by way of the following examples. However, it should be understood that the following examples are solely intended for the purpose of illustration and should not be construed as limiting the disclosure in practice.

### EXAMPLES

### General Procedures:

### 1. Statistical analysis:

The experimental data of all the test groups are expressed as mean ± deviation (SD), and were analyzed using Student's t-test, so as to evaluate the differences between the groups. Statistical significance is indicated by p<0.05.

### Example 1. Evaluation of the effect of composition containing Momordica charantia-derived exosomes and Momordica charantia insulin receptor-binding proteins (mcIRBP) in lowering blood glucose level

### Experimental Materials:

### 1. Experimental mice:

Male ICR mice (4 to 5 weeks old, each weighting approximately 25 g to 35 g) used in the following experiments were purchased from BioLASCO Taiwan Co., Ltd. All the experimental mice were housed in an animal room with an independent air conditioning system under the following laboratory conditions: an alternating 12-hour light and 12-hour dark cycle, a temperature maintained at 22°C ± 2°C, and a relative humidity maintained at 50% to 55%. Furthermore, water and feed were provided *ad libitum* for all the experimental mice. All experimental procedures involving the experimental mice were in compliance with the regulations of the Institutional Animal Care and Use Committee (IACUC) of National Applied Research Laboratories.

### 2. Preparation of Momordica charantia-derived exosomes:

*Momordica charantia* (purchased from a market in Taichung, Taiwan) was chopped, and then double distilled water was added in an amount of 5 mL per 1 gram of the *Momordica charantia,* so as to form a mixture. Subsequently, the mixture was subjected to a homogenization treatment using a homogenizer, followed by centrifugation at 4°C under a speed of 12000 g for 50 minutes using a high-speed centrifuge (KUBOTA; Model: 6500), so as to form a supernatant and a pellet. After that, the supernatant was collected and filtered using a filter membrane having a pore size of 0.22 µm, so as to obtain a filtrate. The filtrate was then subjected to lyophilization, thereby obtaining the *Momordica* charantia-derived exosomes in a powder form.
3. The *Momordica charantia* insulin receptor-binding proteins (mcIRBP) (NCBI Reference Sequence: XP_022137472.1) (SEQ ID NO: 1) used in the following experiments were obtained by entrusting preparation of the same to Tri-I Biotech, Inc.

### 4. Preparation of suspension of composition according to the present disclosure:

The *Momordica* charantia-derived exosomes obtained in Section 2 and the mcIRBP obtained in Section 3 were mixed in a weight ratio of 2:1 to form a composition, followed by suspending the composition in a suitable amount of a carboxymethylcellulose (CMC) aqueous solution containing 0.5 wt% of CMC, so as to obtaining a suspension of the composition.

### Experimental Procedures:

### A. Administration of suspension or CMC aqueous solution:

First, the male ICR mice were randomly divided into three groups, namely, a normal control group, a pathological control group, and an experimental group (n=3 per group). After that, each of the mice in the experimental group was fed, via oral gavage, with the suspension (200 µL) prepared in Section 4 of the *Experimental Materials* of this example at a dose of 1 g/kg body weight. In addition, each of the mice in the normal control group and the pathological control group was fed, via oral gavage, with 200 µL of a CMC aqueous solution containing 0.5 wt% of CMC. Each mouse was fed once a day for 5 days.

### B. Oral glucose tolerance test:

At the end of the 5^{th} day after starting administration of the suspension or the CMC aqueous solution containing 0.5 wt% of CMC, the mice in each group were subjected to oral glucose tolerance test conducted with reference to the method described in Andrikopoulos S. et al. (2008), Am. J. Physiol. Endocrinol. Metab., 295(6):E1323-32. First, the mice in each group were subjected to fasting for a time period ranging from 4 hours to 6 hours. Thereafter, each of the mice in the experimental group and the pathological control group was subjected to intraperitoneal injection with 200 µL of a glucose solution at a dose of 0.5 g/kg body weight, and each of the mice in the normal control group was subjected to intraperitoneal injection with 200 µL of a physiological saline. Prior to the intraperitoneal injection (i.e., at the 0^{th} minute), and at each of the 30^{th}, 60^{th}, 90^{th}, 120^{th} and 180^{th} minute after the intraperitoneal injection, a respective one of the mice in each group was subjected to tail venipuncture, so as to obtain blood samples thereof. In addition, each of the blood samples obtained at the aforesaid time points was subjected to measurement of blood glucose concentration using a blood glucose monitoring system (Smartest), so as to determine blood glucose concentrations (mg/dL) at those time points, respectively.

The data thus obtained were analyzed according to the procedures as described in Section 1 of the *General Procedures,* and then the resultant values were plotted against the abovementioned time points, thereby obtaining a blood glucose concentration versus time curve of each group, as shown in the Figure.

### Results:

Referring to the Figure, during the time period from the 0^{th} minute to the 30^{th} minute after the intraperitoneal injection with the 200 µL of the glucose solution, the blood glucose level of the mice in the pathological control group rose rapidly within this time period, while the blood glucose level of the mice in the experimental group rose relatively slowly, indicating that the composition containing the *Momordica* charantia-derived exosomes and the mcIRBP showed the effect of lowering blood glucose level within 30 minutes after the intraperitoneal injection with the 200 µL of the glucose solution. From the 30^{th} minute to the 180^{th} minute after the intraperitoneal injection with the 200 µL of the glucose solution, the blood glucose level of the mice in the experimental group dropped rapidly within this time period. In addition, the blood glucose concentration of the mice in the experimental group measured at each of the 30^{th}, 60^{th}, 90^{th}, 120^{th} and 180^{th} minute was significantly lower than the blood glucose concentration of the mice in the pathological control group measured at the corresponding time point. In particular, the blood glucose concentration of the mice in the experimental group decreased to below 150 mg/dL within 90 minutes after the intraperitoneal injection with the 200 µL of the glucose solution, whereas the blood glucose concentration of the mice in the pathological control group decreased to approximately 150 mg/dL within 120 minutes after the intraperitoneal injection with the 200 µL of the glucose solution. These results demonstrate that the composition containing the *Momordica charantia*-derived exosomes and the mcIRBP has an excellent blood sugar-lowering effect.

In the description above, for the purposes of explanation, numerous specific details have been set forth in order to provide a thorough understanding of the embodiment(s). It will be apparent, however, to one skilled in the art, that one or more other embodiments may be practiced without some of these specific details. It should also be appreciated that reference throughout this specification to "one embodiment," "an embodiment," an embodiment with an indication of an ordinal number and so forth means that a particular feature, structure, or characteristic may be included in the practice of the disclosure. It should be further appreciated that in the description, various features are sometimes grouped together in a single embodiment, figure, or description thereof for the purpose of streamlining the disclosure and aiding in the understanding of various inventive aspects; such does not mean that every one of these features needs to be practiced with the presence of all the other features. In other words, in any described embodiment, when implementation of one or more features or specific details does not affect implementation of another one or more features or specific details, said one or more features may be singled out and practiced alone without said another one or more features or specific details. It should be further noted that one or more features or specific details from one embodiment may be practiced together with one or more features or specific details from another embodiment, where appropriate, in the practice of the disclosure.

## Claims

1. A composition containing a *Momordica* charantia-derived exosome and a *Momordica charantia* insulin receptor-binding protein for use in lowering blood glucose level in a subject in need thereof.

2. The composition as claimed in claim 1, wherein a weight ratio of the *Momordica* charantia-derived exosome to the *Momordica charantia* insulin receptor-binding protein ranges from 2:1 to 5:1.

3. The composition as claimed in any one of claims 1 and 2, which is a food product or a pharmaceutical composition.

4. The composition as claimed in claim 3, wherein the pharmaceutical composition further contains a pharmaceutically acceptable carrier.

5. The composition as claimed in any one of claims 3 and 4, wherein the pharmaceutical composition is in a dosage form selected from the group consisting of an oral dosage form, a parenteral dosage form, and a topical dosage form.
